# EUROPEAN PATENT APPLICATION

(11) **EP 3 747 363 A1**
(43) Date of publication of application: **09.12.2020**
(21) Application number: 20739552.6
(22) Date of filing: 18.03.2020
(51) Int. Cl.: A61B 5/1455

(54) **NONINVASIVE INTELLIGENT BLOOD GLUCOSE METER**

(30) Priority: 21.03.2019 CN 201910217403
(71) Applicant: Deng, Qingping, Suzhou, Jiangsu 215123 (CN)
(72) Inventor: Deng, Qingping, Suzhou, Jiangsu 215123 (CN)
(74) Representative: Schorr, Peter Karl
(86) International application number: PCT/CN2020/079934
(87) International publication number: WO 2020/187240

(57) **Abstract**

The present invention discloses a noninvasive smart glucometer, comprising a near-infrared picture acquisition device and a processor, wherein the near-infrared picture acquisition device is used for acquiring human body's near-infrared pictures, and the processor is used for processing, comparing and calibrating the near-infrared pictures and then outputting the measurement result of user's blood glucose. While using this glucometer, a blood glucose value can be acquired only by putting the near-infrared pictures of human body's specific parts into the processor. This glucometer is easy to use, and may be shared by multiple people, without any consumables or pollutions, which is a real "noninvasive" glucometer. (Fig. 1a)

## Description

### Technical Field

The present invention relates to a glucometer, and specifically, relates to a noninvasive smart glucometer.

### Background

As we all know, there are billions of diabetics and potential diabetics worldwide. Diabetes has become a very serious problem influencing people's health and life quality. Diabetics need to prevent chronic and acute complications of the disease from time to time. Therefore, diabetics need to measure blood glucose many times every day. Furthermore, because of diabetes pathology's peculiarity that it is no longer reversible once the blood glucose exceeds an index value to become a diabetic. Therefore, even if the person is a potential diabetic, he/she also needs to measure his/her own blood glucose value frequently to prevent becoming a "permanent" diabetic. At present, the commonly used glucometers need to pierce the finger to acquire the blood to be tested, which causes pain and inconvenience and further requires a new test strip for the next test, or there exactly exist instruments that could monitor blood glucose index frequently or continuously, which, however, require the users to conduct subcutaneous implantation about every two weeks and may cause trauma or pain to the users during implantation. The users using these methods all have poor experience, and suffer from financial pressure because of consumables. There are also some so-called "noninvasive" glucometers at present. Although they don't need users to acquire blood using traditional methods for blood glucose detection, they still need users to use traditional methods to acquire blood to establish user's own model corresponding to the blood glucose index value, so they are not true noninvasive glucometers.

Thus, there is a need to provide a truly noninvasive glucometer.

### Summary

According to one main aspect of the present invention, there is provided a noninvasive smart glucometer, which comprises a near-infrared picture acquisition device and a processor,
wherein the near-infrared picture acquisition device is used for acquiring human body's near-infrared pictures, and
the processor is used for processing, comparing and calibrating the near-infrared pictures and then outputting a measurement result of user's blood glucose.

Preferably, the noninvasive smart glucometer further comprises an input-output device for the user's manipulation and displaying information. Preferably, the noninvasive smart glucometer further comprises an external device and an interface communicating with the external device, the external device communicating with other components of the noninvasive smart glucometer in a wired and/or wireless way.

Preferably, the near-infrared picture acquisition device is a finger near-infrared picture acquisition device for acquiring near-infrared pictures of a user's finger.

Preferably, the near-infrared picture acquisition device comprises a near-infrared camera, a near-infrared light source component and a limb fixing device.

Preferably, the finger near-infrared picture acquisition device comprises a near-infrared camera, a near-infrared light source component and a finger fixing device.

Preferably, the near-infrared light source component comprises one or more sets of near-infrared lamps emitting near-infrared light with different wavelengths.

Preferably, the near-infrared light source component comprises three sets of near-infrared lamps emitting near-infrared light with different wavelengths. Preferably, the near-infrared picture acquisition device sequentially acquires three sets of near-infrared pictures with different wavelengths.

Preferably, the wavelength of near-infrared light emitted by the near-infrared light source component ranges from 700nm to 1800nm.

Preferably, a first wavelength of near-infrared light emitted by the near-infrared light source component ranges from 700nm to 800nm, a second wavelength ranges from 800nm to 900nm, and a third wavelength ranges from 900nm to 1000nm.

Preferably, the first wavelength of near-infrared light emitted by the near-infrared light source component ranges from 750nm to 800nm, the second wavelength ranges from 850nm to 900nm, and the third wavelength ranges from 950nm to 1000nm.

Preferably, the near-infrared camera is disposed on one side of the finger fixing device, and the near-infrared light source component is disposed on the other side of the finger fixing device.

Preferably, the near-infrared camera is disposed under the finger fixing device, and the near-infrared light source component is disposed above the finger fixing device.

Preferably, the near-infrared camera and the finger fixing device are separated by transparent glass.

Preferably, the finger fixing device comprises a U-shaped groove. Preferably, the position of the U-shaped groove corresponding to a fingertip is provided with a switch for starting the acquisition of near-infrared pictures of the finger.

Preferably, the switch is a touch-sensitive switch.

Preferably, the bottom of the U-shaped groove is hollow.

Preferably, the processor comprises a picture processing module and a comparison and calibration module.

Preferably, the processor further comprises a data management module. Preferably, the picture processing module processes the acquired near-infrared pictures and then transmits the pictures to the comparison and calibration module for comparison and calibration.

Preferably, the picture processing comprises associating the acquired near-infrared pictures with the wavelength of near-infrared light at the time of shooting.

Preferably, the comparison and calibration module is obtained through training on a computer with powerful computation capability by applying artificial intelligence machine learning algorithm and a large amount of training data.

Preferably, the artificial intelligence machine learning algorithm is deep learning algorithm.

Preferably, the computer with powerful computation capability is a GPU computer.

Preferably, the training data comprises a finger near-infrared light picture of a person to be acquired and a corresponding blood glucose index, and the wavelength of the near-infrared light corresponding to the finger near-infrared light picture used in the training data is the same as the wavelength of the near-infrared light used by the finger near-infrared picture acquisition device.

Preferably, the comparison and calibration module is set to be able to operate normally under the condition without a network.

Preferably, the data management module is used for managing user's information and analyzing historical data of the user's blood glucose value as measured.

The noninvasive smart glucometer according to the present invention can acquire finger's near-infrared pictures and transmit these pictures to the comparison and calibration module to obtain user's blood glucose index directly and in real time, without blood samples or blood stain and trauma, and there is no tingling or risk of infection or cross-infection for the user. Moreover, the use of the noninvasive smart glucometer of the present invention demands no consumables and produces no disposable products or hazardous wastes, the user's usage frequency is not limited, and the cost of use will not be added, and accordingly, the financial pressure of diabetics will be reduced so that their life quality will be improved and also, the potential diabetics will be reminded to prevent themselves to become "permanent" diabetics. In addition, the noninvasive smart glucometer of the present invention is convenient to carry and easy to operate. It can be used anytime and anywhere regardless of whether it is at home or in an office or other public places, which greatly facilitates users. Moreover, the noninvasive smart glucometer of the present invention can record historical results of the user to help the doctor analyze, study and develop treatment. One instrument may also be used by multiple persons or the whole family, without causing mutual infection or the confusion of data results. These advantages have all greatly improved user's experience and life quality.

### Brief Description of Drawings

By reading in conjunction with the drawings, the overview above and following detailed description will be better understood. For the sake of illustration, the drawings show some embodiments as disclosed. However, it shall be understood that the present invention is not limited to the precise arrangements and tools as shown. The accompanying drawings incorporated into and forming part of this description illustrate the implementation of the system and device according to the present invention, and together with the description, describe the advantages and principles according to the present invention.
Fig. 1a schematically shows a functional framework diagram of a noninvasive smart glucometer according to one embodiment of the present invention;
Fig. 1b schematically shows the structure of the noninvasive smart glucometer in Figure 1a;
Fig. 2 schematically shows a flow chart of the use of a noninvasive smart glucometer according to one embodiment of the present invention.

### Embodiments

Before detailedly describing at least one embodiment of the present invention, it shall be understood that the present invention is not limited to the details of the structure and the application of the component arrangement described below or shown in the drawings. The drawings and written description are provided to guide persons skilled in the art to carry out and use the present invention for which patent protection is sought. The present invention is applicable to other embodiments and can be implemented and executed in various ways. Persons skilled in the art will understand that, for clarity and ease of understanding, not all features of one commercial embodiment are shown. Those skilled in the art will also understand that the development of actual commercial embodiments incorporating the aspects of the present invention will require implementation of many specific judgements to achieve the ultimate goal of the developer's commercial embodiments. Although these tasks are complicated and time-consuming, these tasks are routine for those skilled in the art that benefit from the present disclosure.

Moreover, it should be understood that the wording and terms used herein are for ease of description and should not be regarded as limitations. For example, the use of singular terms, such as "a" or "an", is not intended to limit the number of items. In addition, the use of non-limiting relational terms, such as "top", "bottom", "left", "right", "above", "below", "upward", "downward" and "side", is for the sake of clarity, not intended to limit the scope of the present invention or the appended claims. Furthermore, it should be understood that any of the features of the present invention may be used alone or used in combination with other features. By reading the drawings and the detailed description, those skilled in the art will clearly understand other systems, methods, features and advantages of the present invention. It is expected that all such other systems, methods, features and advantages are included in this description, included within the scope of the present invention, and protected by the appended claims.

The implementation of the present invention will now be described in detail with reference to the accompanying drawings.

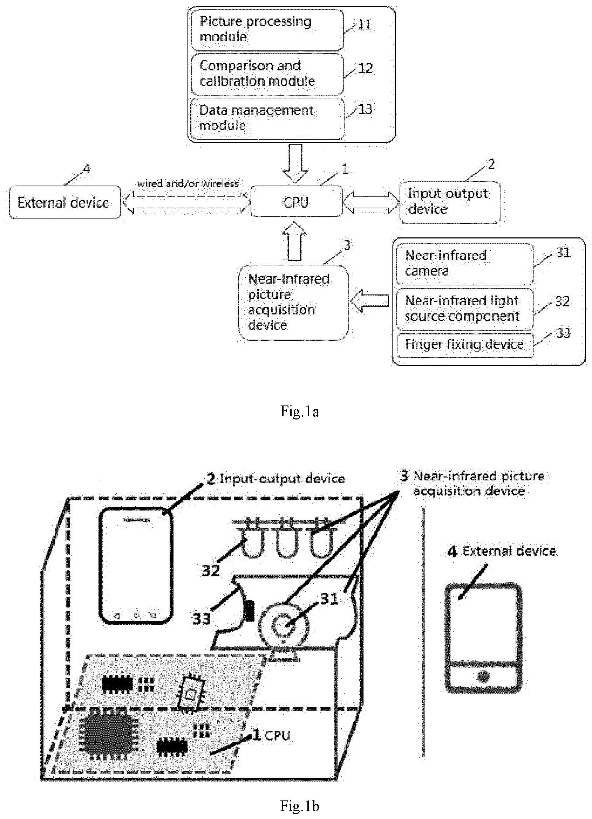

Referring to Fig. 1a, in one embodiment of the present invention, a noninvasive smart glucometer 100 comprises a processor 1, an input-output device 2, a finger near-infrared picture acquisition device 3, and an external device 4, wherein the finger near-infrared picture acquisition device acquires near-infrared pictures of a user's finger (pictures taken with a near-infrared camera under near-infrared light), then these pictures are transmitted to the processor 1 for processing, and a result is outputted after comparison and calibration completed in the processor 1. The result may be displayed in the input-output device 2 integrated into the noninvasive smart glucometer 100, and may also be transmitted to the external device 4.

It shall be known that the input-output device 2 and the external device 4 may not be included in other embodiments of the present invention. In this embodiment they are described together for the purpose of detail, but this does not mean that these components are necessary.

As shown in Fig. 1a, the finger near-infrared picture acquisition device 3 comprises a near-infrared camera 31, a near-infrared light source component 32, and a finger fixing device 33. The near-infrared camera 31 is used for shooting finger's near-infrared pictures, and the near-infrared light source component 32 is used for emitting near-infrared light to user's finger to facilitate the shooting of the near-infrared camera 31. When the user puts the finger correctly on the finger fixing device 33, the near-infrared light source component 32 sequentially emits three different wavelengths of near-infrared light, and the near-infrared camera 31 sequentially takes three near-infrared pictures with different wavelengths. It should be understood that although the near-infrared light is preferably used in this embodiment, it is also allowable to use light of other frequency bands in spectrum in other embodiments, and these alternatives should not be excluded out of the scope of the present invention. It should also be understood that although it is preferable to acquire pictures of three different wavelengths of near-infrared light in this embodiment, it is also allowable in other embodiments to use pictures with one, two, or four, five or even more different wavelengths, and these alternatives should also not be excluded out of the scope of the present invention.

Referring to Fig. 1b, the near-infrared light source component 32 is positioned above the finger fixing device 33, and the near-infrared camera 31 is positioned under the finger fixing device 33. The finger fixing device 33 and the near-infrared camera 31 are separated by transparent glass, which plays the role of dust prevention for the near-infrared camera 31 and through which the near-infrared camera 31 could take a picture for the finger. It is emphasized again that the words "above" and "under" in this embodiment are just for indicating the relative position between components, and in other embodiments, for example, the near-infrared light source component 32 may be positioned on the left side of the finger fixing device 33, and the near-infrared camera 31 may be positioned on the right side of the finger fixing device 33. Of course, it is preferred that such positions of one side and the other side are directly opposite, but in some implementation forms, it is also allowed to deviate from the directly opposite positions to a certain extent. Persons skilled in the art should understand that as long as the near-infrared light source component 32 can irradiate the user's finger and the near-infrared camera 31 can take pictures for the finger passed through by the near-infrared light, these solutions shall not be excluded out of the protection scope of the present invention.

The finger fixing device 33 is a "U-shaped" groove with a hollow bottom. The top of the "U-shape" (the position corresponding to the fingertip) is provided with a capacitive touch-sensitive switch, the function of which is triggered by a capacitive sensing chip circuit installed there. It should be understood that, in other embodiments, the finger fixing device 33 may not be U-shaped but may be any other suitable shapes; its bottom may not be necessarily hollow, and it is permitted as long as the near-infrared camera 31 can take pictures for the finger passed through by the near-infrared light; and the touch-sensitive switch does not have to be set on the top of the U-shape but may be set at any other suitable position. It also should be understood that although the touch-sensitive switch is capacitive in this embodiment, other forms of touch-sensitive switches are also allowable. Moreover, even if the switch is in another form, such as mechanical switch, photosensitive switch or voice switch, they are all feasible. These alternatives shall not be excluded out of the protection scope of the present invention.

When the top of the fingertip touches the switch on the top, the process of shooting near-infrared pictures will be started. The near-infrared light source component 32 comprises three sets of near-infrared lamps, wherein each set includes one or more near-infrared lamps, and preferably each set includes 8-12 near-infrared lamps. Each set of near-infrared light may emit the near-infrared light with the wavelength of this set when it is turned on. When the near-infrared camera 31 starts to shoot, the respective sets of near-infrared lamps with different wavelengths are turned on in sequence, and the finger's near-infrared pictures are taken at the wavelengths in sequence. Specifically, the near-infrared light source component 32 turns on the near-infrared light of the first wavelength, the near-infrared camera 31 takes the finger's near-infrared picture at the first wavelength, then the near-infrared light source component 32 turns on the near-infrared light of the second wavelength, the near-infrared camera 31 takes the finger's near-infrared picture at the second wavelength, and then the near-infrared light source component 32 turns on the near-infrared light of the third wavelength, the near-infrared camera 31 takes the finger's near-infrared picture at the third wavelength. In a preferred embodiment, the entire photographing process takes less than 1 second. After the picture is taken, the user may remove the finger from the finger fixing device 33 by himself. It should be understood that, in some embodiments, the aforementioned light-emitting and photographing process can be completed automatically once started, while in other embodiments, the process can also be controlled by the user, for example, by means of the aforementioned switch or an additionally arranged switch. It should also be understood that, in some embodiments, after the photographing is finished, the user may be prompted by light or sound, for example, by the input-output device 2 or external device 4 of the noninvasive smart glucometer 100. Of course, a warning lamp or loudspeaker may also be set for reminding. These alternatives should not be excluded out of the protection scope of the present invention. The three different wavelengths of the near-infrared light source component 32 all range from 700nm to 1800nm. In the preferred embodiment, the first wavelength ranges from 700nm to 800nm, the second wavelength ranges from 800nm to 900nm, and the third wavelength ranges from 900nm to 1000nm. In a more preferred embodiment, the first wavelength ranges from 750nm to 800nm, the second wavelength ranges from 850nm to 900nm, and the third wavelength ranges from 950nm to 1000nm.

As shown in Fig. 1a, it is preferred that the processor 1 is a CPU processor, comprising a picture processing module 11, a comparison and calibration module 12 and a data management module 13. The picture processing module 11 processes the near-infrared pictures acquired by the near-infrared picture acquisition device 3 and then transmits the pictures to the comparison and calibration module 12 for comparison and calibration, and the result of comparison and calibration module 12 is output to the data management module 13, where the data is analyzed and then output to the input-output device 2 or the external device 4 for a user.

In the picture processing module 11, the near-infrared pictures as acquired are associated with the near-infrared wavelength at the time of shooting, and the pictures are compared and calibrated based on the specific wavelength information in the comparison and calibration module 12. In the preferred embodiment, the comparison and calibration module 12 reflects the corresponding blood glucose index through three near-infrared pictures with different wavelengths, and obtains the final blood glucose index upon comparison and calibration. In some embodiments, the program for controlling the photographing of the finger's near-infrared picture acquisition device 3 may also be integrated into the picture processing module 11. Of course, the function of the picture processing module 11 may also be integrated into the comparison and calibration module 12 or into the finger's near-infrared picture acquisition device 3 directly.

In the preferred embodiment, the comparison and calibration module 12 is obtained through training using artificial intelligence machine learning algorithm, such as deep learning algorithm, which transmits the processed near-infrared pictures to the comparison and calibration module 12 to obtain a corresponding user's blood glucose value. Specifically, the comparison and calibration module 12 is obtained through training on a computer with powerful computation capability, such as a GPU computer, using deep learning algorithm based on a large amount of original data acquired specially, which is taken as basic training data upon accurate sorting and matching. The original data comprises finger's near-infrared pictures of a person to be acquired and corresponding blood glucose indexes. The wavelength of the near-infrared light corresponding to the finger's near-infrared pictures used in the training data is consistent with the wavelength of near-infrared light used in the finger's near-infrared picture acquisition device 3. For example, the finger's near-infrared pictures in the training data are also acquired using three different wavelengths ranging from 700nm to 1800nm. Preferably, three different wavelengths ranging from 700nm to 800nm, ranging from 800nm to 900nm, and ranging from 900nm to 1000nm are used. More preferably, three different wavelengths ranging from 750nm to 800nm, ranging from 850nm to 900nm and ranging from 950nm to 1000nm are used.

In the preferred embodiment, the blood glucose index values corresponding to the acquired training data are distributed between 3.6 and 22.0 with the span of 0.1. As for each blood glucose value, at least 40 pictures are acquired under every wavelength. Of course, it should be understood that the distribution and span of other numerical ranges and the number of the acquired pictures are adjustable. These alternatives should not be excluded out of the protection scope of the present invention.

In the preferred embodiment, the trained model (engine) is embedded into the comparison and calibration module 12, and the user's blood glucose value may be calculated directly after user's near-infrared pictures are transmitted. So the user doesn't need to acquire blood using traditional methods for blood glucose detection, nor does the user need to use other non-purely noninvasive glucometers to acquire blood by traditional methods to establish the user's own model corresponding to the blood glucose index value at the initial phase. Meanwhile, the trained model also guarantees that the glucometer of the present invention can work without a network, that is, all works of measuring, calculating and storing may be completed locally in the glucometer without any cloud intervention (such as cloud storage and cloud computing). Of course, the glucometer of the present invention does not reject cooperation with a cloud device.

The data management module 13 is used for managing user's information, and analyzing historical data of user's measured blood glucose value. The data management module 13 may comprise a user information registration submodule for registering user's basic information including user's name, age, gender, home address and other additional specification. Preferably, many users may be registered by means of the user information registration submodule. The data management module 13 may also comprise a historical data analyzing submodule, by which a historical database of blood glucose values may be established for each of the users, and this historical database may be output in an appropriate chart form to the input-output device 2 and/or external device 4 as feedback for the user. Meanwhile, in light of the currently measured data and historical data variation, corresponding healthcare advice may further be given to the user, which advice may also be output to the input-output device 2 and/or external device 4 as feedback for the user.

It should be understood that, in other embodiments of the present invention, some or all of the data management module 13 may be excluded. For the purpose of comprehensiveness, they are described together, but it does not mean that these modules are necessary.

As shown in Fig. 1b, the input-output device 2 is a piece of touch screen, which is installed on the main body of the noninvasive smart glucometer 100 together with the processor 1 and the finger's near-infrared picture acquisition device 3. The input-output device 2 is used for inputting editing and presenting every user's personal information and status of the measured blood glucose level, historical data, analyzed result and healthcare advices. If there is a measurement result, the input-output device 2 may output the result directly or together with the historical data and/or healthcare advice according to user's setting. If there is no measurement result, the input-output device 2 may provide a prompt to the user to measure again. It should be understood that the input-output device 2 may be a capacitive or resistive touch screen, and in other embodiments, the input-output device 2 may also be a traditional form of an untouchable screen plus a physical button or an input-output form using voice. These alternatives should not be excluded out of the protection scope of the present invention.

The external device 4 may be user's own cellphone, Pad computer or PC. In the preferred embodiment, it communicates with the processor 1 in a wired or wireless way, and the noninvasive smart glucometer 100 is provided with a corresponding interface or communication module for communicating with the external device 4. If necessary, corresponding APP program or API program may also be used. The wireless way includes, for example, WiFi, Bluetooth, and Zigbee, etc, and also includes, for example, 3G, 4G and 5G, etc. Of course, the external device 4 may also be a component produced and sold with other components of the noninvasive smart glucometer 100, just independent of the main body of the noninvasive smart glucometer 100. Persons skilled in the art should understand that if the aforesaid input-output device 2 is also set out of the main body of the noninvasive smart glucometer 100, in some certain sense, the input-output device 2 may also be deemed as an external device. Persons skilled in the art should also understand that the input-output device 2 and the external device 4 are not mutually exclusive but may be used simultaneously. These alternatives should not be excluded out of the protection scope of the present invention.

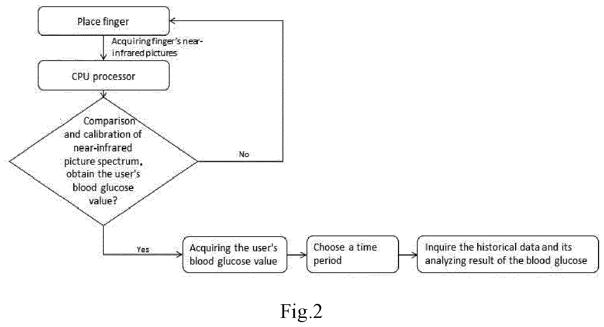

Referring to Fig. 2, it schematically shows a flow chart of use of a noninvasive smart glucometer according to one embodiment of the present invention. As the figure shows, the user firstly places a finger on a finger fixing device 33 of a finger's near-infrared picture acquisition device 3, triggers a switch to start acquiring finger's near-infrared pictures, and then the acquired pictures are transmitted to a CPU processor 1, in which comparison and calibration of near-infrared picture spectrum is performed after processing, so as to obtain the user's blood glucose value. If the blood glucose value of the user cannot be acquired after the CPU processor 1's processing, the user shall place the finger again to conduct acquiring and analyzing. After acquiring the user's blood glucose value, the user may also choose a time period to inquire the historical data and its analyzing result of the blood glucose value during this time period.

It should be understood that in the aforesaid embodiments, the present invention is illustrated by taking the acquisition and analysis of a finger near-infrared picture as an example, but the present invention is not limited to only using user's finger. Other appropriate methods, such as shooting and analyzing palm or other parts of human body, are also feasible. These alternatives should not be excluded out of the protection scope of the present invention.

In addition, persons skilled in the art should understand that because of diversity of combination of software and hardware (including firmware), one software function is not necessarily implemented only by one hardware, and more than one software function may be integrated into one hardware for implementation, and may also be dispersed in more than one hardware for implementation. Unless otherwise specified, hardware's separation or integration does not restrict the software function. Particularly, because of the development of cloud storage and cloud computation technology, some functions may be not only realized locally, but also realized on "Cloud", or both. Unless otherwise specified, these alternatives should not be excluded out of the protection scope of the present invention.

Persons skilled in the art will understand that modifications can be made to the above-mentioned embodiments without departing from its broad summary of the invention. Therefore, it should be understood that the present invention disclosed herein is not limited to the specific embodiments as disclosed, but is intended to cover all variations within the spirit and scope of the present invention as defined by the appended claims. In addition, even if the spirit and scope of the present invention defined by the appended claims are interpreted according to the embodiments disclosed in the present invention, unless the applicant makes special instructions, the principle of donation should not be used to evaluate the spirit and scope of the present invention.

## Claims

1. A noninvasive smart glucometer, comprising a near-infrared picture acquisition device and a processor,
wherein the near-infrared picture acquisition device is used for acquiring human body's near-infrared pictures, and the processor is used for processing, comparing and calibrating the near-infrared pictures and then outputting a measurement result of user's blood glucose.

2. The noninvasive smart glucometer according to claim 1, further comprising an input-output device for the user's manipulation and displaying information.

3. The noninvasive smart glucometer according to claim 1, further comprising an external device and an interface communicating with the external device, the external device communicating with other components of the noninvasive smart glucometer in a wired and/or wireless way.

4. The noninvasive smart glucometer according to claim 1, **characterized in that** the near-infrared picture acquisition device is a finger near-infrared picture acquisition device for acquiring near-infrared pictures of a user's finger.

5. The noninvasive smart glucometer according to claim 4, **characterized in that** the finger near-infrared picture acquisition device comprises a near-infrared camera, a near-infrared light source component and a finger fixing device.

6. The noninvasive smart glucometer according to claim 5, **characterized in that** the near-infrared light source component comprises one or more sets of near-infrared lamps emitting near-infrared light with different wavelengths.

7. The noninvasive smart glucometer according to claim 5, **characterized in that** the near-infrared light source component comprises three sets of near-infrared lamps emitting near-infrared light with different wavelengths.

8. The noninvasive smart glucometer according to claim 7, **characterized in that** the near-infrared picture acquisition device sequentially acquires three sets of near-infrared pictures with different wavelengths.

9. The noninvasive smart glucometer according to claim 5, **characterized in that** the wavelength of near-infrared light emitted by the near-infrared light source component ranges from 700nm to 1800nm.

10. The noninvasive smart glucometer according to claim 5, **characterized in that** the near-infrared camera is disposed on one side of the finger fixing device, and the near-infrared light source component is disposed on the other side of the finger fixing device.

11. The noninvasive smart glucometer according to claim 5, **characterized in that** the near-infrared camera and the finger fixing device are separated by transparent glass.

12. The noninvasive smart glucometer according to claim 5, **characterized in that** the finger fixing device comprises a U-shaped groove, and
the position of the U-shaped groove corresponding to a fingertip is provided with a switch for starting the acquisition of near-infrared pictures of the finger.

13. The noninvasive smart glucometer according to claim 1, **characterized in that** the processor comprises a picture processing module and a comparison and calibration module,
wherein the picture processing module processes the acquired near-infrared pictures and then transmits the pictures to the comparison and calibration module for comparison and calibration, and
the picture processing comprises associating the acquired near-infrared pictures with the wavelength of near-infrared light at the time of shooting.

14. The noninvasive smart glucometer according to claim 13, **characterized in that** the comparison and calibration module is obtained through training on a computer with powerful computation capability by applying artificial intelligence machine learning algorithm and a large amount of training data, and
the training data comprises a finger near-infrared light picture of a person to be acquired and a corresponding blood glucose index, and the wavelength of the near-infrared light corresponding to the finger near-infrared light picture used in the training data is the same as the wavelength of the near-infrared light used by the finger near-infrared picture acquisition device.

15. The noninvasive smart glucometer according to claim 14, **characterized in that** the comparison and calibration module is set to be able to operate normally under the condition without a network.
